# EUROPEAN PATENT APPLICATION

(11) **EP 3 432 310 A1**
(43) Date of publication of application: **23.01.2019**
(21) Application number: 18177702.0
(22) Date of filing: 14.06.2018
(51) Int. Cl.: G16H 20/40, G16H 50/50

(54) **METHOD AND SYSTEM FOR A PREOPERATIVE SURGICAL INTERVENTION SIMULATION**

(30) Priority: 17.07.2017 RU 2017112089
(71) Applicant: ENSIM LLC ("ENSIM" Limited Liability Company), Kazan (RU)
(72) Inventor: GORBUNOV, Maksim Anatolievich, 420087, KAZAN, Republic of Tatarstan (RU); NIKOLAEV, Denis Nikolaevich, 420047 KAZAN,Republic of Tatarstan (RU)
(74) Representative: Glawe, Delfs, Moll

(57) **Abstract**

The technical solution relates to the medical sphere and is designed for preoperative planning and performance of a rehearsal surgical intervention, laparoscopic nephrectomy in particular, using a 3D patient's model created on base of the patient's data. Technical result of use of the present solution is to provide preoperative planning and performance of a rehearsal laparoscopic surgical intervention as well as to perform a training session for surgeons in a more accurate way. This is being achieved by generating a 3D model of abdominal cavity; the 3D model comprises coordinates, size and location of organs inside the abdominal cavity of an actual patient. The preoperative simulation of a surgical intervention is characterized in that:
- Data on patient's examination with use of a contrast agent are being obtained;
- Following parameters (location, trajectory and size of the great vessels and of vessel groups flowing around the abdominal cavity organs) is being distinguished and specified from the said data by filtrating examination data array in a density range corresponding to blood vessels;
- A map of the blood vessels is being generated on the base of the said parameters;
- A map of the patient's abdominal cavity is being generated by correlating location, trajectory and size of the great vessels and of vessel groups flowing around the abdominal cavity organs with coordinates, size and location of organs inside the abdominal cavity of the patient on the base of the map of the blood vessels;
- A 3D model of the patient's abdominal cavity is being generated by scaling a template 3D model of a patient on the base of the map of the patient's abdominal cavity;
- The 3D model of the patient's abdominal cavity is being transferred and used for the upcoming of the rehearsal surgical intervention simulation.

## Description

### TECHNICAL FIELD

The technical solution relates to the medical sphere and is designed for preoperative planning and performance of a rehearsal surgical intervention, laparoscopic nephrectomy in particular, using a 3D patient's model created on base of the patient's data.

### BACKGROUND ART

For transplantation planning it is very important to give a surgeon a visual information on the organ blood vessels' structure, their exact location inside the organ and what organ areas are being flown around by one or another vessel.

**Preoperative Surgical Simulation** US8500451 (B2**)** technical solution, published on **06.08.2013,** by **SIMBIONIX LTD** is known. This solution discloses an apparatus and a simulation method for rehearsal vascular surgical intervention (angioplasty) using 3D model created on the base of medical images of a real patient. The method includes obtaining, by an input system, a 3D model of vessels for an actual patient created on the base of the patient's data array (CT); rehearsal surgical intervention simulation using the 3D model of the patient's vessels, wherein the simulation system receives and processes the signals, received from the interventional instruments imitators motion detectors and signals to the force feedback mechanism.

This invention is designed for preoperative planning and rehearsal vascular surgical intervention that renders it impossible to use it for preoperative planning and rehearsal laparoscopic surgical intervention. Inability of rehearsal laparoscopic surgical intervention performance is supposed to be a defective feature of the said technical solution.

**SYSTEM AND METHOD FOR PERFORMING COMPUTERIZED SIMULATIONS FOR IMAGE-GUIDED PROCEDURES USING A PATIENT SPECIFIC MODEL** US8543338 (B2**),** published on **24.09.2013,** by **SIMBIONIX LTD** is known.

This solution discloses a method and a simulation system of rehearsal vascular surgical intervention (angioplasty) using a digital model of anatomical structures created on the base of medical images of a real patient. This method includes producing a digital model of anatomical structures in a patient based on medical image data obtained from the patient, where the digital model includes a first 3D polygonal mesh representing the medical image data; based on the image-based data producing of adjacent anatomical regions; producing the extended model comprising anatomical structures of the image-based data, adjacent anatomical regions obtained by generating; the first 3D polygonal mesh; the second 3D polygonal mesh obtained from the adjacent anatomical regions; positioning the second 3D polygonal mesh in alignment with a boundary of the first 3D polygonal mesh; performing a computerized simulation of an image-guided procedure using the extended model.

This solution is designed for preoperative planning and rehearsal vascular surgical intervention that renders it impossible to use it for preoperative planning and rehearsal Laparoscopic surgical intervention.

### SUMMARY OF THE TECHNICAL SOLUTION

The present technical solution is designed for removal of disadvantages of the existing prior art.

Technical result of use of the present solution is to provide preoperative planning and performance of a rehearsal laparoscopic surgical intervention as well as to perform a training session for surgeons in a more accurate way.

This technical result is being achieved by generating a 3D model of abdominal cavity; the 3D model comprises coordinates, size and location of organs inside the abdominal cavity of an actual patient.

One of the preferred embodiments of the preoperative simulation of a surgical intervention is characterized in that:
- Data on patient's examination with use of a contrast agent are being obtained;
- Following parameters (location, trajectory and size of the great vessels and of vessel groups flowing around the abdominal cavity organs) is being distinguished and specified from the said data by filtrating examination data array in a density range corresponding to blood vessels;
- A map of the blood vessels is being generated on the base of the said parameters;
- A map of the patient's abdominal cavity is being generated by correlating location, trajectory and size of the great vessels and of vessel groups flowing around the abdominal cavity organs with coordinates, size and location of organs inside the abdominal cavity of the patient on the base of the map of the blood vessels;
- A 3D model of the patient's abdominal cavity is being generated by scaling a template 3D model of a patient on the base of the map of the patient's abdominal cavity;
- The 3D model of the patient's abdominal cavity is being transferred and used for the upcoming of the rehearsal surgical intervention simulation.

Rehearsal surgical intervention simulation with use of the obtained 3D model of the patient's abdominal cavity may include choice of optimal ports on the abdominal cavity surface.

Rehearsal surgical intervention simulation with use of the obtained 3D model of the patient's abdominal cavity may include colon mobilization for obtaining an exposure to the retroperitoneum where the kidneys are located; clipping and transection of the ureter, renal artery and vein, kidney mobilization and its extraction.

Distinguishing and specification of the patient's examination data may include delimitation of external boundaries of the patient's abdominal cavity; initial and terminal key points specification; these points are being used for the scaling of a template 3D model of a patient.

This technical solution can be embodied in a form of the system for a preoperative surgical intervention simulation comprising:
- At least one instruction control unit;
- At least one data storage unit;
- One or more software programs downloaded to at least one said data storage unit and run at least one said instruction control unit; assuming one or more said software programs contain the following instructions:
   ○ Data on patient's examination with use of a contrast agent are being obtained;
   ○ Following parameters (location, trajectory and size of the great vessels and of vessel groups flowing around the abdominal cavity organs) is being distinguished and specified from the said data by filtrating examination data array in a density range corresponding to blood vessels;
   ○ A map of the blood vessels is being generated on the base of the said parameters;
   ○ A map of the patient's abdominal cavity is being generated by correlating location, trajectory and size of the great vessels and of vessel groups flowing around the abdominal cavity organs with coordinates, size and location of organs inside the abdominal cavity of the patient on the base of the map of the blood vessels;
   ○ A 3D model of the patient's abdominal cavity is being generated by scaling a template 3D model of a patient on the base of the map of the patient's abdominal cavity;
   ○ The 3D model of the patient's abdominal cavity is being transferred and used for the upcoming of the rehearsal surgical intervention simulation.

Rehearsal surgical intervention simulation with use of the obtained 3D model of the patient's abdominal cavity may include choice of optimal ports on the abdominal cavity surface.

Rehearsal surgical intervention simulation with use of the obtained 3D model of the patient's abdominal cavity may include colon mobilization for obtaining an exposure to the retroperitoneum where the kidneys are located; clipping and transection of the ureter, renal artery and vein, kidney mobilization and its extraction.

Distinguishing and specification of the patient's examination data may include delimitation of external boundaries of the patient's abdominal cavity; initial and terminal key points specification; these points are being used for the scaling of a template 3D model of a patient.

### BRIEF DESCRIPTION OF THE DRAWINGS

**FIG.1** is an image of the great vessels obtained with use of CT with intravenous contrast enhancement;
**FIG.2** is an image of the vessels groups of the certain organs;
**FIG.3** is a system for the method for a preoperative surgical intervention simulation embodiment;
**FIG.4** is a flowchart for one of the options for the method for a preoperative surgical intervention simulation embodiment.

### DETAILED DESCRIPTION OF THE TECHNICAL SOLUTION

In this device the system is understood as a computer system, an ECM (electronic computing machine), a CNC (computer numeric control), a PLC (programmable logic controller), computerized control systems and any other devices capable of performing a given, clearly defined sequence of operations (actions, instructions).

The instruction unit is understood as an electronic unit or an integrated circuit (microprocessor) executing machine instruction (programs).

The instruction unit reads and executes the instructions (programs) from one or more storage devices. Storage device can be represented, but is not limited to, hard drives (HDD), flash memory, ROM (read-only memory), solid state drives (SSD), optical drives (CD, DVD, Blue-Ray discs).

Program is a sequence of instruction designed for execution by the computing machine control device or instruction unit.

Some terms used for the description of the technical solution are listed below:

**Laparoscopy** is a modern surgical technique when an operation performed through small incisions (usually 0.5-1.5 cm) in comparison to an open procedure with greater incisions.

**Radiocontrast agents** (syn. contrast agents) are substances used to enhance the visibility of invisible or blurred internal structures (organs or cavities) in X-ray-based imaging techniques. The effect of the radiocontrast agents is based on a considerable increase of the X-ray deposition difference between examined anatomical structures and surrounding tissues.

Computed tomography is a method of nondestructive layer-by-layer examination of the internal structure of a subject. The method is based on measurement and complex computer processing of the difference of x-rays attenuation with tissues of a different density. Now, x-ray computed tomography is the main method of research of internal organs examination with usage of x-rays.

Voxel (originated by analogy with the word "pixel", with vo representing "volume" and el representing "element") is an element of a 3D image containing raster item value in a three-dimensional space. Voxels are analogous to 2D pixels but in the three-dimensional space. Voxel models are often used for medical and scientific information visualization and analysis.

The present technical solution provides means for planning and performance of a rehearsal laparoscopic intervention by virtue of the creation of an abdominal cavity 3D model with coordinates, size and location of organ within the abdominal cavity of the actual patient as well as well as for performing a training session for surgeons in a more accurate way.

The method for a preoperative surgical intervention simulation comprises the following steps according to the present technical solution:
**Data on patient's examination with use of a contrast agent are being obtained.**

Detailed patient information can be obtained by examination, for example computed tomography (CT), magnetic resonance imaging (MRI), etc.

Modern tomographic scanner save the examination data in a special format - DICOM. DICOM file contains information on the intensity or density of tissues in a certain cross section, in each cross section point. Cross sections may be performed in three planes: sagittal, frontal and horizontal. DICOM files combine in series and are a set of the sequential cross sections of an organ or body area.

We shall call the data in the series and all the points (voxels) of each cross section in the series an examination data array, representing a 3D array, where each element stores point coordinates and tissue density. Downloading, processing, and use of the information stored in DICOM files do not involve a certain technical difficulty according to the current background.

CT saves radiodensity in files. This radiodensity depends on a tissue physical density. For quantitative describing of the radiodensity the Hounsfield scale is used; tissue density range is starting from -1024 HU up to +3071 HU. Average indices according to the Hounsfield scale are: air is -1000 HU, fat is -120 HU, water is 0 HU, soft tissues are +40 HU, bones are +400 HU and higher.

**Following parameters (location, trajectory and size of the great vessels and of vessel groups flowing around the abdominal cavity organs) is being distinguished and specified from the said data by filtrating examination data array in a density range corresponding to blood vessels**

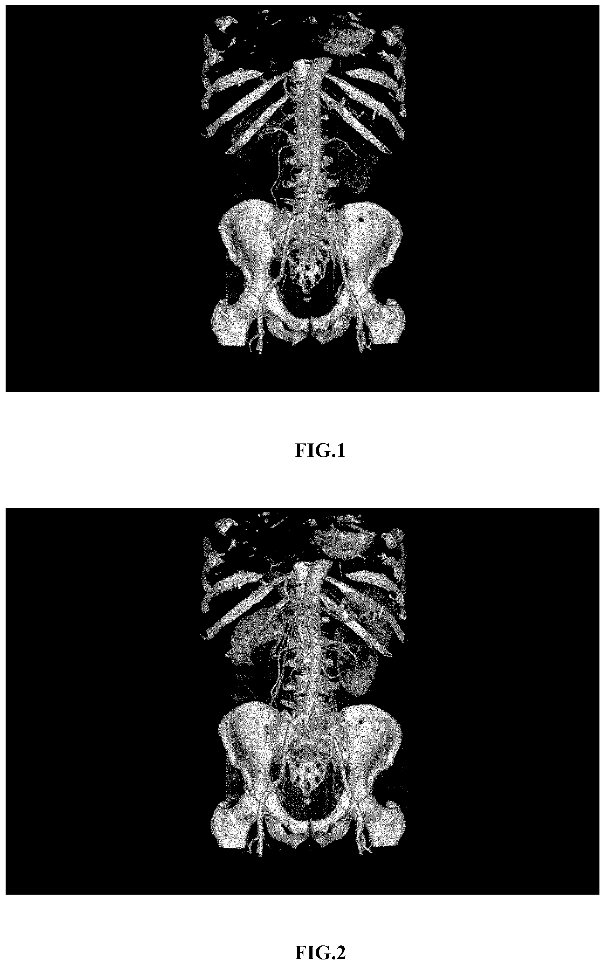

CT does not render it possible to distinguish between the organs automatically as the internals have almost the same density, being a main problem occurring by the patient's 3D model generation on the base of the CT images. There is an intravenous contrast computed tomography helping to increase "visibility" of arterial and venous vessels for X-ray; with that vessel density according to the Hounsfield scale is being increased. This method is used in CT angiography and helps to measure vessel lumen, presence of blood clots, aneurysms, and vascular wall thinning. Intravenous contrast allows marking out the vessel information from the general CT image array. **FIG.1** represents the image of great vessels obtained with the intravenous contrast computed tomography.

**A map of the blood vessels is being generated on the base of the said parameters.**

This step is being performed by creating a vessels model skeleton. The vessels model skeleton is a set of interrelated data stored the information on the vessels trajectory and vessel's width in each trajectory point. Principle of the vessels model skeleton creation on the base of examination array is described in detail in 3D Liver Vessels Model Design Using CT Data by Artem M. Yatcheniko, Andrey S. Krylov, Andrey V.Gavrilov and Ivan V. Arkhipov (Conference Proceedings of the 19th International Conference on Computer Graphics and Vision "GraphiCon'2009", Moscow, 2009).

At first, vessel data are being detached by filtration the data array in density range appropriate to the vessels. After data array binarization the vessel on the cross section are represented in the form of the simple connected scopes of points, at the same time if two adjacent cross sections are superimposed, then scopes of points of one and the same vessel partially overlap each other.

Overlap degree depends on the cross section pace taken during the examination; this pace could start from 0.5 mm. Partial overlap of the scopes of points of the vessel on the adjacent cross sections renders it possible to form the vessel trajectory; number of points in the scopes of points of the vessel allows to calculate an approximate vessel width (diameter); presence of the common scopes of points of different vessels (when the vessels separate or group together) renders it possible to define the vessels connection and to create the vessels model skeleton.

**A map of the patient's abdominal cavity is being generated by correlating location, trajectory and size of the great vessels and of vessel groups flowing around the abdominal cavity organs with coordinates, size and location of organs inside the abdominal cavity of the patient on the base of the map of the blood vessels.**

Human's blood vessels are branching. Great vessels have a greater diameter than those that are bringing the blood the organs. The vessels model skeleton allows calculating the great vessels and branches of supplying and emissary vessels on the base of the vessel width in each trajectory point. A doctor (or an operator in a semiautomatic mode) recognizes the branches of vessels relative to a certain organ in the vessels model skeleton in one of the embodiments of the said technical solution.

A group of vessels flowing around an organ is regarded to as a set of vessels in area of a certain organ. The set of vessels is calculated from each vessels branch belonging to the organ; at that vessels with the least width and outermost vessels in each vessels branch are included to the group. The group of vessels flowing around an organ contains the information on the organ coordinates, its size (volume), and spatial orientation.

**FIG.2** represents the vessels groups of the certain organs.

**A 3D model of the patient's abdominal cavity is being generated by scaling a template 3D model of a patient on the base of the map of the patient's abdominal cavity.**

Scaling of the patient's template 3D model abdominal cavity comprises resizing of the 3D model of the abdominal cavity, reshaping of the 3D model of the abdominal cavity in accordance with the patient's body.

Resizing of the 3D model of the abdominal cavity can be performed according to two key points being defined by an operator in the examination data array. At that, the 3D model of the abdominal cavity shall contain corresponding key points. For example, an upper and lower points of the lumbar spine could be taken as the key points. It is possible to calculate the distance between the key points and to scale the 3D model of the abdominal cavity in accordance with the calculated distance being aware of the image resolution and the distance between the cross sections in a series.

In one of the embodiments of the said technical solution, the key points are set by a doctor (or an operator) on the base of the examination data, for example marking an initial and a terminal points during the data visualization.

Patient's examination data store information on the patient's abdominal cavity external boundaries. Points on the external boundaries of the abdominal cavity have a density different from the density of air, border on points with air density, and are located as close as possible to the external boundary of the cross section. The points set on the abdominal cavity external boundary determines the shape of the patient's body and is being used to change the shape of the 3D models of the abdominal cavity in accordance with the shape of the patient's body.

Thus, the map of the patient's abdominal cavity is being generated on the base of the examination data array. This map comprises the cavity size, coordinates, size (volume), special orientation of the organs, vessel model skeleton comprising vessels trajectory and their width in each trajectory point.

The template 3D model of the patient includes 3D model of the abdominal cavity and 3D models of the organs.

In one of the embodiments of the said technical solution, the scaling of the patient's template 3D model in accordance with a map of the abdominal cavity of the patient includes the following steps:
Scaling of the abdominal cavity accordance to the size of the abdominal cavity of the patient'
Scaling and positioning of the organs;
Generating of great vessels in accordance with the trajectory and size of the vessels.

Alternatively, scaling of the patient's template 3D model in accordance with a map of the abdominal cavity of the patient may additionally include a choice of template organs models, for example: models with any defects, diseases.

Scaling of the patient's template 3D model in accordance with a map of the abdominal cavity of the patient is generating of the 3D models of the abdominal cavity with the organs and blood vessels, arrangement of which is similar to a real one in the actual patient, takes place as a result.

**The 3D model of the patient's abdominal cavity is being transferred and used for the upcoming of the rehearsal surgical intervention simulation.**

In one of the embodiments of the said technical solution, preoperative surgical intervention simulation is being performed by means of laparoscopic simulators making it possible to simulate the intervention, wherein a surgeon is performing an intervention in virtual reality using the medical instruments imitators.

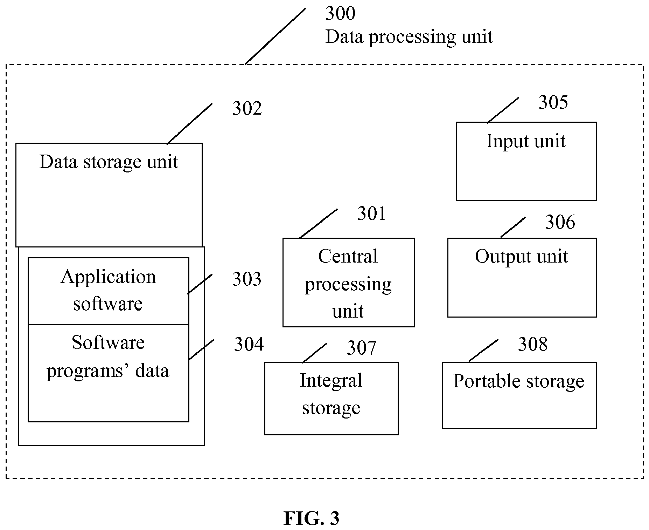

According to **FIG.3****,** an approximate system for the said technical solution embodiment includes data processing unit 300. The data processing unit 300 may be configured as a client, a server, a mobile device or any other computing device interacting with the data within a collaborative work system based on the net.

In the basic configuration the data processing unit 300 includes as a rule at least one central processing unit (CPU) 301 and data storage unit 302. In relation to the exact configuration and computing device type, the data storage unit 302 may be volatile (for example, random access memory (RAM)), nonvolatile (for example, read-only memory (ROM)) or their combination. As a rule, data storage unit 302 comprises one or more application software and may include software programs' data 304. The said technical solution as a method described in details above is embodied in the application software 303.

The data processing unit 300 may possess extra features or functionalities, for example, the data processing unit 300 may include extra data storage devices (portable and integral) like magnetic disks, optical disks or tape. Such extra storages are presented on FIG.3 in the form of integral storage 307 and portable storage 308. Computer data storages may include volatile, nonvolatile, integral and portable data storages implemented in any way or by means of any technology for data storage. The data storage unit 302, the integral storage 307 and the portable storage 308 are examples of the computer data storages. The computer data storages comprise, but are not limited to, a random access memory (RAM), a read-only memory (ROM), an electrically erasable programmable read-only memory (EEPROM), a flash drive or memory storages embodied using any other technology, a compact disc ROM (CD-ROM), a digital versatile disc (DVD) or other optical memory, tape cassettes, magnetic tapes, storages on the magnetic discs or other magnetic memory storages, or any other environment that may be used for the data storage and to which the data processing unit 300 can gain an access. Any such computer data storage can be a part of the data processing unit 300. The data processing unit 300 may also include input unit (units) 305, such as a keyboard, a mouse, a stylus, a voice command device, a touch-input device, etc. Output unit (units) 306, such as a display, dynamics, a printer and such, may also be included to the unit.

The data processing unit 300 has communication connection making it possible for the unit to connect other computing device, for example by network. Networks comprise local area networks and global network as well as the other great scalable networks including, but are not limited to, corporate network and extranets.

The communication connection is an example of the communication medium. As a rule, the communication medium may be embodied by means of machine-readable instructions, data structures, program modules, or any other data in a modulated information signal, such as a carrier wave or any other transfer mechanism, and includes any medium of the information delivery. The modulated information signal means a signal with one or more characteristics changed or set in a way to code the information in this signal. For example, but not limited to, the communication media include a wired medium, such as a wired network or a direct wired connection, and wireless medium, such as acoustic, RF, infrared and other wireless media. The term "machine-readable medium" as used in this document includes both storage media and communication environment.

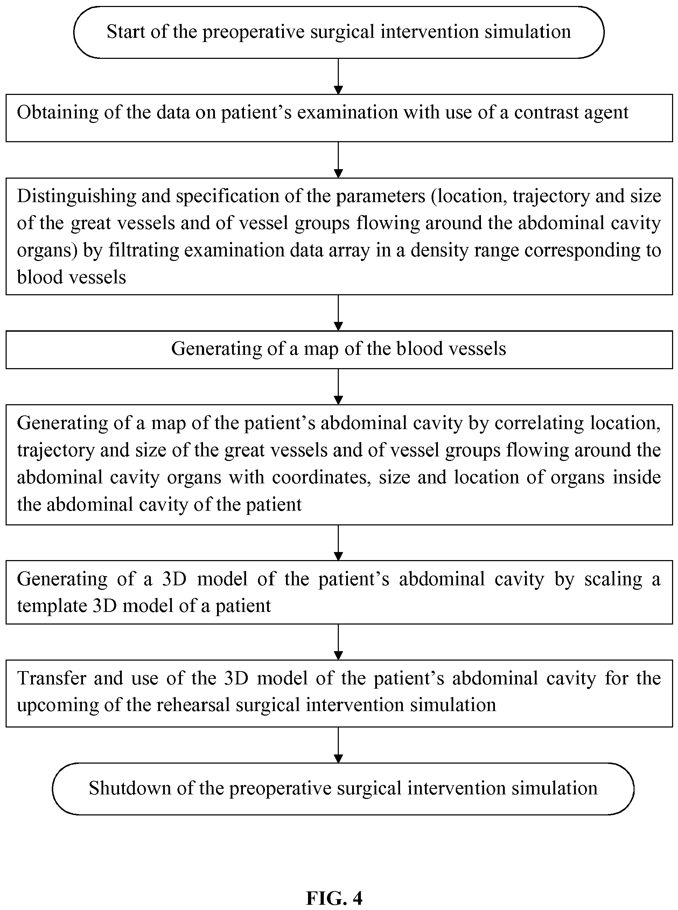

FIG.4 represents a flowchart for one of the options for the method for a preoperative surgical intervention simulation embodiment

It is evident for experts that the given options for the method and the system for a preoperative surgical intervention simulation embodiment are described here to illustrate the solution; various embodiments are not going beyond the scope of the invention.

## Claims

1. A method for a preoperative simulation of a surgical intervention, wherein
• Data on patient's examination with use of a contrast agent are being obtained;
• Following parameters (location, trajectory and size of the great vessels and of vessel groups flowing around the abdominal cavity organs) is being distinguished and specified from the said data by filtrating examination data array in a density range corresponding to blood vessels;
• A map of the blood vessels is being generated on the base of the said parameters;
• A map of the patient's abdominal cavity is being generated by correlating location, trajectory and size of the great vessels and of vessel groups flowing around the abdominal cavity organs with coordinates, size and location of organs inside the abdominal cavity of the patient on the base of the map of the blood vessels;
• A 3D model of the patient's abdominal cavity is being generated by scaling a template 3D model of a patient on the base of the map of the patient's abdominal cavity;
• The 3D model of the patient's abdominal cavity is being transferred and used for the upcoming of the rehearsal surgical intervention simulation.

2. The method of claim 1, wherein the rehearsal surgical intervention simulation with use of the obtained 3D model of the patient's abdominal cavity includes choice of optimal ports on the abdominal cavity surface.

3. The method of claim 1, wherein the rehearsal surgical intervention simulation with use of the obtained 3D model of the patient's abdominal cavity includes colon mobilization for obtaining an exposure to the retroperitoneum where the kidneys are located; clipping and transection of the ureter, renal artery and vein, kidney mobilization and its extraction.

4. The method of claim 1, wherein distinguishing and specification of the patient's examination data include delimitation of external boundaries of the patient's abdominal cavity; initial and terminal key points specification; these points are being used for the scaling of a template 3D model of a patient.

5. The system for a preoperative surgical intervention simulation comprising:
- At least one instruction control unit;
- At least one data storage unit;
- One or more software programs downloaded to at least one said data storage unit and run at least one said instruction control unit; assuming one or more said software programs contain instructions for the method embodiment according to any of the claims 1-4.

6. Machine-readable medium containing executing machine-readable instructions by one or more data processing unit; execution of the said instructions embodies the method embodiment according to any of the claims 1-4.
